# EUROPÄISCHE PATENTANMELDUNG

(11) **EP 1 122 247 A1**
(43) Veröffentlichungstag der Anmeldung: **08.08.2001**
(21) Anmeldenummer: 00102543.6
(22) Anmeldetag: 07.02.2000
(51) Int. Cl.: C07D 301/12

(54) **Verfahren zur Epoxidierung von Olefinen**

(71) Anmelder: Degussa AG, 40474 Düsseldorf (DE)
(72) Erfinder: Hofen, Willi, 63517 Rodenbach (DE); Thiele, Georg, Dr., 63452 Hanau (DE)
(74) Vertreter: Sternagel, Fleischer, Godemeyer & Partner Patentanwälte

(57) **Zusammenfassung**

Die hierin beschriebene Erfindung betrifft ein Verfahren zur katalytischen Epoxidierung von Olefinen mit Wasserstoffperoxid in einem kontinuierlich durchströmten Reaktionssystem, wobei in dem Reaktionssytem eine gasförmige Phase, die ein Olefin enthält und eine flüssige Phase, die das Wasserstoffperoxid enthält, vorliegt und die gasförmige Phase im Gegenstrom zur flüssigen Phase geführt wird.

## Beschreibung

### Stand der Technik

Aus EP-A 100 119 ist bekannt, daß sich Propen mit Wasserstoffperoxid zu Propenoxid umsetzen läßt, wenn als Katalysator ein titanhaltiger Zeolith eingesetzt wird.

Unumgesetztes Wasserstoffperoxid läßt sich aus der Reaktionsmischung der Epoxidierung nicht wirtschaftlich zurückgewinnen. Darüber hinaus verursacht nicht umgesetztes Wasserstoffperoxid zusätzlichen Aufwand bei der Aufarbeitung der Reaktionsmischung. Deshalb wird die Epoxidierung von Propen bevorzugt mit einem Propenüberschuß und bis zu einem hohem Wasserstoffperoxidumsatz geführt. Für das Erzielen eines hohen Wasserstoffperoxidumsatzes ist die Verwendung eines kontinuierlich durchströmten Reaktionssystems von Vorteil. Solch ein Reaktionssystem kann entweder aus einem oder mehreren Strömungsrohrreaktoren bestehen oder aus einer Anordnung von 2 oder mehr durchmischten Reaktoren, die in Reihe geschaltet sind. Beispiele für durchmischte Reaktoren sind Rührkessel, Schlaufenreaktoren, Wirbelbettreaktoren und Festbettreaktoren mit einer Rückführung der flüssigen Phase.

Um eine hohe Reaktionsgeschwindigkeit zu erreichen ist eine möglichst hohe Propenkonzentration in der flüssigen Phase erforderlich. Die Reaktion wird deshalb vorteilhaft unter Propenatmosphäre bei erhöhtem Druck durchgeführt.

Als Nebenreaktion tritt am Titansilicalitkatalysator stets in geringem Umfang die Zersetzung von Wasserstoffperoxid unter Bildung von molekularem Sauerstoff auf. Um den Epoxidierungsprozeß im technischen Maßstab sicher betreiben zu können muß der gebildete Sauerstoff aus dem Reaktionssystem entfernt werden. Dies geschieht am einfachsten durch Ausschleusung mit einem Propenabgasstrom.

EP-A 659 473 beschreibt ein Epoxidierungsverfahren, daß diese Elemente vereinigt. Dabei wird eine flüssige Mischung aus Wasserstoffperoxid, Lösungsmittel und Propen über eine Folge von in Reihe geschalteten Festbettreaktionszonen geleitet, wobei aus jeder Reaktionszone die flüssige Phase entnommen wird, zur Entfernung der Reaktionswärme über einen externen Wärmetauscher geleitet wird und dann zum überwiegenden Teil in dieselbe Reaktionszone zurückgeführt wird und ein geringerer Teil der flüssigen Phase in die nächste Zone geleitet wird. Die einzelnen Reaktionszone verhalten sich wegen der Flüssigkeitsrückführung über das Festbett als durchmischte Reaktoren. Gleichzeitig wird mit der flüssigen Einsatzstoffmischung gasförmiges Propen eingespeist, im Parallelstrom zur flüssigen Phase über die Festbettreaktionszonen geführt und am Ende des Reaktionssystems neben der flüssigen Reaktionsmischung als sauerstoffhaltiger Abgasstrom entnommen. Diese Reaktionsführung ermöglicht zwar eine Erhöhung der Propenoxidausbeute im Vergleich zu herkömmlichen Rohrreaktoren ohne die in EP-A 659 473 beschriebene Temperaturkontrolle, führt aber aufgrund der Komplexität des für die Ausführung des Verfahrens notwendigen Reaktionssystems zu erheblichen zusätzlichen Kosten. Darüber hinaus ist die beschriebene erhöhte Ausbeute auch nur dann realisierbar, wenn das im Abgasstrom enthaltene Propenoxid zurückgewonnen wird. Dies erzwingt eine zusätzliche Prozeßstufe, was die Kosten des Verfahrens noch zusätzlich erhöht.

Aufgabe der vorliegenden Erfindung ist es daher, ein einfaches kostengünstiges Verfahren zur Epoxidierung von Olefinen mit Wasserstoffperoxid zur Verfügung zu stellen, mit dem hohe Umsätze bei gleichzeitig hoher Produktausbeute erreicht werden kann und das mit herkömmlichen Reaktionssystemen durchgeführt werden kann.

### Gegenstand der Erfindung

Diese Aufgabe wird durch ein Verfahren zur katalytischen Epoxidierung von Olefinen mit Wasserstoffperoxid in einem kontinuierlich durchströmten Reaktionssystem gelöst, wobei in dem Reaktionssystem eine gasförmige Phase, die ein Olefin enthält und eine flüssige Phase, die das Wasserstoffperoxid enthält, vorliegt und die gasförmige Phase im Gegenstrom zur flüssigen Phase geführt wird.

Ein wesentlicher Vorteil der erfindungsgemäßen Gegenstromführung liegt in der Verringerung des Propenoxidaustrags aus dem Reaktionssystem zusammen mit dem sauerstoffhaltigen Propenabgasstrom und in dem dadurch verminderten Aufwand zur Rückgewinnung von Propenoxid aus diesem Abgasstrom. Ein möglichst geringer Verlust an Propenoxid ist anzustreben, um erfindungsgemäß eine hohe Produktausbeute zu erzielen.

Die erfindungsgemäße Gegenstromführung von gasförmigem Olefin und flüssiger Reaktionsmischung im Reaktionssystem kann in Abhängigkeit vom gewählten Reaktionssystem auf unterschiedliche Weise verwirklicht werden. Hierzu sind Reaktionssysteme geeignet, bei denen bezogen auf das Gesamtsystem keine vollständige Rückvermischung stattfindet, d.h. Reaktionssysteme deren Verweilzeitspektrum ein Maximum aufweist oder Reaktionssysteme mit Pfropfströmung.

Wenn die Epoxidierung von Olefinen in einem Strömungsrohrreaktor durchgeführt wird, dann wird der Gasstrom, der das Olefin enthält, innerhalb des Reaktors im Gegenstrom zur flüssigen Phase geführt. Vorzugsweise wird dabei der Flüssigkeitsstrom von oben nach unten durch den Reaktor geführt, während das Olefin den Reaktor von unten nach oben gasförmig durchströmt. Der Reaktor kann dabei sowohl mit einer kontinuierlichen Flüssigphase als Blasensäule, als auch mit einer kontinuierlichen Gasphase als Rieselreaktor betrieben werden. Der Katalysator kann dabei entweder als Suspension in der flüssigen Phase oder in Form eines Festbetts eingesetzt werden, wobei das Festbett sowohl als ungeordnete Katalysatorschüttung, als auch als geordnete Packung aus beschichteten Monolithen oder Verteilerkörpern ausgeführt werden kann. Bevorzugt wird ein Strömungsrohrreaktor als Festbettreaktor mit ungeordneter Katalysatorschüttung und kontinuierlicher Flüssigphase eingesetzt.

Um auch beim Wechsel bzw. der Regenerierung des Epoxidierungskatalysators einen kontinuierlichen Betrieb des Verfahrens zu erzielen, können wahlweise auch zwei oder mehr Strömungsrohrreaktoren in der beschriebenen Weise parallel oder in Reihe betrieben werden.

Wenn die Epoxidierung von Olefin in einer Serie von zwei oder mehr in Reihe geschalteten Strömungsrohrreaktoren durchgeführt wird, können die Stoffströme von flüssiger Phase und gasförmiger Phase innerhalb eines Strömungsrohres entweder im Gleichstrom oder im Gegenstrom geführt werden, wobei die Stoffströme zwischen den Strömungsrohren im Gegenstrom geführt werden.

In einer alternativen Ausführungsform kann das Reaktionssystem mehrere in Reihe geschaltete Reaktoren aufweisen, die unabhängig von einander aus durchmischten Reaktoren und Strömungsrohren ausgewählt sind, wobei die Stoffströme von flüssiger Phase und gasförmiger Phase zwischen den Reaktoren im Gegenstrom geführt werden. Z.B können innerhalb des Reaktionssystems von in Reihe geschalteten Reaktoren auch durchmischte und Strömungsrohrreaktoren in Kombination eingesetzt werden. Bevorzugt werden dabei ein oder mehrere durchmischte Reaktoren mit einem abschließenden Strömungsrohrreaktor in Reihe geschaltet. Der besondere Vorteil eines solchen Reaktionssystems liegt darin, daß sich aus den durchmischten Reaktoren, in denen der größte Teil des Reaktionsumsatzes erfolgt, die Reaktionswärme besonders einfach abführen läßt. Mit dem abschließenden Strömungsrohr ist dann sichergestellt, daß der Wasserstoffperoxidumsatz möglichst vollständig erfolgt. Als durchmischte Reaktoren eignen sich beispielsweise Rührkessel, Schlaufenreaktoren, Strahlreaktoren mit Flüssigkeitsumlauf oder Festbettreaktoren mit einem Flüssigkeitskreislauf über das Festbett.

Mit dem erfindungsgemäßen Verfahren können Olefine epoxidiert werden, die unter den gewählten Reaktionsbedingungen sich zumindest teilweise in der Gasphase befinden. Dies sind insbesondere die Olefine mit 2-6 Kohlenstoffatomen. Ganz besonders gut geeignet ist das erfindungsgemäße Verfahren für die Epoxidierung von Propen zu Propenoxid.

Aus wirtschaftlichen Gründen würde man gern für ein technisches Verfahren Propen nicht in reiner Form sondern als technische Mischung mit Propan einsetzten, die in der Regel 1 bis 15 Vol-% Propan enthält. Da die Epoxidierungsreaktion Propen verbraucht, reichert sich Propan im Gasstrom auf dem Weg durch das Reaktionssystem an, was bei einer Gleichstromführung zu einer Abnahme der Reaktionsgeschwindigkeit und zu Unterschieden in der Wärmeentwicklung durch die exotherme Epoxidierungsreaktion entlang der Reihe der Reaktoren führt. Durch die erfindungsgemäße Gegenstromführung von Gasphase und flüssiger Phase können diese Nachteile vermieden werden. Weiterhin ist die Propenoxidausbeute gerade bei Gegenwart von Propan im Zuführstrom im Vergleich zu einer Gleichstromführung bei der Gegenstromführung der Stoffströme erhöht. Hieraus wird deutlich, daß mit Hilfe des erfindungsgemäßen Verfahrens nicht nur ein hoher Umsatz und hohe Propenoxidausbeute mit geringem apparativem Aufwand erreicht werden kann, sonder auch der Einsatz von technischem Propen mit bis zu 15 % Propan sich nicht nachteilig auf die Reaktionsführung und die Produktausbeute auswirkt. Aufgrund der Verwendbarkeit billigerer Ausgangsmaterialien wird die Wirtschaftlichkeit des erfindungsgemäßen Verfahrens weiter verbessert.

Als Katalysator eignen sich für das erfindungsgemäße Epoxidierungsverfahren kristalline, titanhaltige Zeolithe der Zusammensetzung (TiO₂)ₓ(SiO₂)₁₋ₓ mit x von 0.001 bis 0.05 und einer MFI- bzw. MEL-Kristallstruktur, bekannt als Titansilicalit-1 und Titansilicalit-2. Solche Katalysatoren können z.B. nach dem in US-A 4,410,501 beschriebenen Verfahren hergestellt werden. Der Titansilicalitkatalysator kann als Pulver oder als verformter Katalysator in Form von Granulaten, Extrudaten oder Formkörpern eingesetzt werden. Zur Formgebung kann der Katalysator 1 bis 99 % eines Bindemittels oder Trägermaterials enthalten, wobei alle Bindemittel und Trägermaterialien geeignet sind, die unter den zur Epoxidierung angewandten Reaktionsbedingungen nicht mit Wasserstoffperoxid oder dem Epoxid reagieren. Als Suspensionskatalysatoren werden bevorzugt Granulate entsprechend EP-A 893 158 eingesetzt. Als Festbettkatalysatoren werden bevorzugt Extrudate mit einem Durchmesser von 1 bis 5 mm eingesetzt.

Das Wasserstoffperoxid wird bei dem erfindungsgemäßen Verfahren in Form einer wässrigen Lösung mit einem Wasserstoffperoxidgehalt von 1 bis 90 Gew.-%, bevorzugt von 10 bis 70 Gew.-% und besonders bevorzugt von 30 bis 50 Gew.-% eingesetzt. Das Wasserstoffperoxid kann in Form der im Handel erhältlichen, stabilisierten Lösungen eingesetzt werden. Ebenso geeignet sind nicht stabilisierte, wässrige Wasserstoffperoxidlösungen, wie sie bei dem Anthrachinonverfahren zur Herstellung von Wasserstoffperoxid erhalten werden.

Die Reaktion wird bevorzugt in Gegenwart eines Lösungsmittels durchgeführt, um die Löslichkeit des Olefins vorzugsweise des Propens in der flüssigen Phase zu erhöhen. Als Lösungsmittel geeignet sind alle Lösungsmittel, die unter den gewählten Reaktionsbedingungen nicht oder nur in geringem Maß durch Wasserstoffperoxid oxidiert werden und sich mit mehr als 10 Gew.-% in Wasser lösen. Bevorzugt werden Lösungsmittel, die mit Wasser unbegrenzt mischbar sind. Geeignete Lösungsmittel sind Alkohole wie z.B. Methanol, Ethanol oder tert-Butanol; Glykole wie z.B. Ethylenglykol, 1,2-Propandiol oder 1,3-Propandiol; cyclische Ether wie z.B. Tetrahydrofuran, Dioxan oder Propylenoxid; Glykolether wie z.B. Ethylenglykolmonomethylether, Ethylenglykolmonoethylether, Ethylenglykolmonobutylether oder die Propylenglykolmonomethylether und Ketone wie z.B. Aceton oder 2-Butanon. Besonders bevorzugt wird Methanol als Lösungsmittel zugesetzt.

Das erfindungsgemäße Verfahren zur Epoxidierung von Olefinen vorzugsweise Propen wird bei einer Temperatur von -10 bis 100°C, vorzugsweise bei 20 bis 70°C durchgeführt. Das Olefin wird vorzugsweise im Überschuß zu Wasserstoffperoxid eingesetzt um einen weitgehenden Wasserstoffperoxidumsatz zu erreichen, wobei das molare Verhältnis von Olefin vorzugsweise Propen zu Wasserstoffperoxid vorzugsweise im Bereich von 1.1 bis 10 gewählt wird. Bei Zusatz eines Lösungsmittels wird die Lösungsmittelmenge vorzugsweise so gewählt, daß in der Reaktionsmischung nur eine flüssige Phase vorliegt. Bevorzugt wird das Lösungsmittel in einem Gewichtsverhältnis von 0.5 bis 20 relativ zur eingesetzten Wasserstoffperoxidlösung zugesetzt. Die eingesetzte Katalysatormenge kann in weiten Grenzen variiert werden und wird vorzugsweise so gewählt, daß unter den angewandten Reaktionsbedingungen innerhalb von 1 min bis 5 h ein Wasserstoffperoxidumsatz von mehr als 90 %, vorzugsweise mehr als 95 % erreicht wird.

Bei einer bevorzugten Ausführungsform des erfindungsgemäßen Verfahrens wird Propen eingesetzt, das zwischen 0 und 15% Propan enthalten kann. Propen kann sowohl als Flüssigkeit als auch in Gasform in das Reaktionssystem eingespeist werden. Die eingespeiste Propenmenge wird dabei so gewählt, daß sich unter den Reaktionsbedingungen in den Reaktoren eine Gasphase bildet, die im wesentlichen aus Propen besteht und daß aus dem ersten Reaktor des Reaktionssystems ein Abgas entnommen werden kann, dessen Sauerstoffgehalt außerhalb der Explosionsgrenzen für Propylen-Sauerstoff-Mischungen liegt. Der Druck im Reaktionssystem wird vorzugsweise zwischen 50 und 100 % des Sättigungsdampfdrucks von Propylen bei der Reaktionstemperatur gewählt.

Im folgenden soll die vorliegende Erfindung anhand von Figuren und Beispielen für die Epoxidierung von Propen veranschaulicht werden.

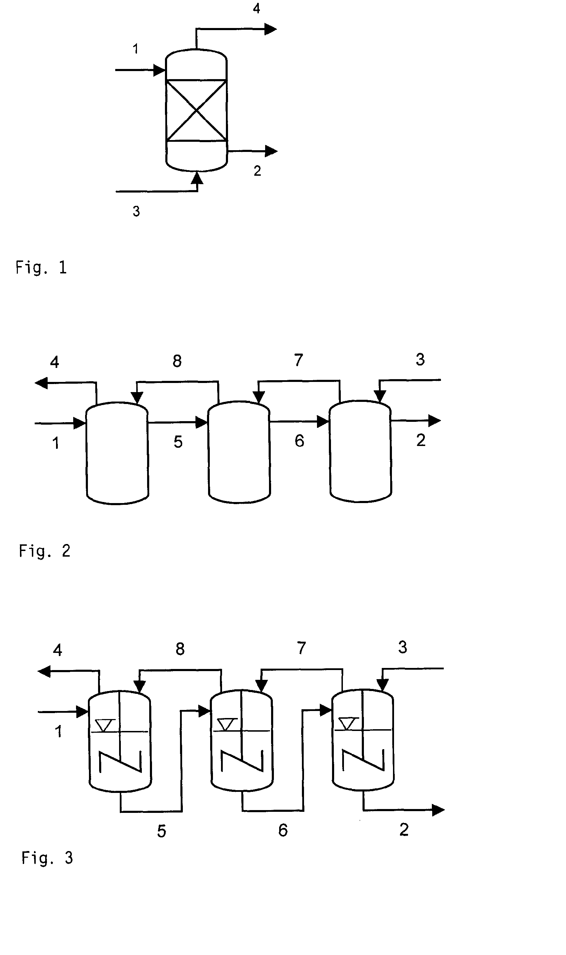

Fig. 1 zeigt einen Strömungsrohrreaktor. Strom 1 bezeichnet den Zuführstrom der flüssigen Reaktionsphase, Strom 2 bezeichnet den Austrittsstrom der flüssigen Reaktionsphase, Strom 3 bezeichnet den Zuführstrom der gasförmigen Komponente und Strom 4 bezeichnet den Abgasstrom. Diese Bezeichnungen werden auch für die weiteren Figuren beibehalten.

Fig. 2 illustriert die Führung der Stoffströme für drei in Reihe geschaltete Reaktoren. Die flüssigen Einsatzstoffe werden mit Strom 1 in den ersten Reaktor eingespeist. Vom ersten Reaktor wird die flüssige Reaktionsmischung über die Ströme 5 und 6 in den zweiten und dritten Reaktor geführt und aus dem dritten Reaktor als Strom 2 in flüssiger Form entnommen. Mit dem Strom 3 wird Propen, gegebenenfalls in Mischung mit Propan, in den dritten Reaktor eingespeist und mit den Strömen 7 und 8 gasförmig über den zweiten Reaktor in den ersten Reaktor geleitet. Aus dem ersten Reaktor wird über den Strom 4 ein Abgasstrom entnommen, der neben nicht umgesetztem Propen und gegebenenfalls Propan den während der Epoxidierungsreaktion durch Zersetzung von Wasserstoffperoxid gebildeten molekularen Sauerstoff enthält.

Fig. 3 zeigt als Beispiel ein System aus drei in Reihe geschalteten Rührkesseln für die Epoxidierung mit einem Suspensionskatalysator, das in der erfindungsgemäßen Gegenstromführung von Flüssigphase und Propengas betrieben wird, wobei die Numerierung der Stoffströme mit Fig. 2 übereinstimmt.

Wenn Strömungsrohrreaktoren in Reihe geschaltet werden, kann die Führung der Stoffströme innerhalb eines Reaktors sowohl im Gegenstrom als auch im Gleichstrom erfolgen.

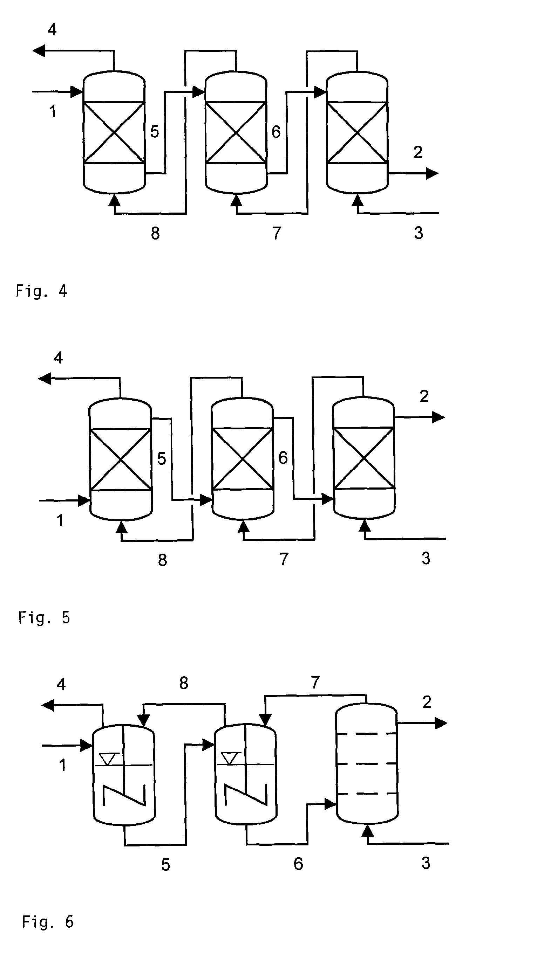

Fig. 4 zeigt als Beispiel ein System aus drei in Reihe geschalteten Festbettreaktoren mit Gegenstromführung innerhalb der Reaktoren, wobei die Stoffströme von Flüssigphase und Propengas zwischen den Reaktoren in der erfindungsgemäßen Weise im Gegenstrom geführt werden.

Fig. 5 zeigt als Beispiel ein System aus drei in Reihe geschalteten Festbettreaktoren mit Gleichstromführung innerhalb der Reaktoren, wobei die Stoffströme von Flüssigphase und Propengas zwischen den Reaktoren in der erfindungsgemäßen Weise im Gegenstrom geführt werden. In beiden Abbildungen stimmt die Numerierung der einzelnen Stoffströme mit Fig. 2 überein.

Fig. 6 zeigt als Beispiel die Kombination von zwei Rührkesseln mit einem im Gleichstrom betriebenen Blasensäulenreaktor für die Epoxidierung mit einem Suspensionskatalysator, wobei die Stoffströme von Flüssigphase und Propylengas zwischen den Reaktoren in der erfindungsgemäßen Weise im Gegenstrom geführt werden. Die Numerierung der einzelnen Stoffströme stimmt dabei mit Fig. 2 überein.

### Beispiel:

In einer Anordnung aus zwei Rührkesseln und einem Strömungsrohrreaktor mit einem Gesamtvolumen von 6.25 1, die entsprechend Fig. 6 untereinander verbunden sind, werden in den ersten Reaktor parallel 43 Gew.-% Wasserstoffperoxid mit 1045 g/h und eine 2.0 Gew.-% Suspension von Titansilicalit in Methanol mit 2630 g/h eingespeist (Strom 1). Gleichzeitig werden in den dritten Reaktor von unten 1120 g/h Propen gasförmig eingespeist (Strom 5). Die drei Reaktoren werden auf 65°C thermostatisiert und der Druck in allen drei Reaktoren wird durch ein Druckhalteventil am ersten Reaktor auf einem Überdruck von 15.0 bar gehalten. Am Druckhalteventil werden 215 g/h nichtumgesetztes Propen mit einem Sauerstoffgehalt von 0.6 Vol-% entnommen (Strom 8). In regelmäßigen Abständen wird in der aus dem dritten Reaktor entnommenen flüssigen Reaktionsmischung (Strom 4) der Wasserstoffperoxidgehalt durch Redoxtitration und der Gehalt an Propenoxid, 1-Methoxy-2-propanol, 2-Methoxy-1-propanol und 1,2-Propandiol durch GC bestimmt. Nach Erreichen des stationären Betriebszustands liegt der Wasserstoffperoxidumsatz bei 96,8 %, die Propenoxidausbeute bezogen auf umgesetztes Wasserstoffperoxid bei 90,3 % und die Propenoxidselektivität, berechnet als das Verhältnis der Konzentration von Propenoxid zur Summe der Konzentrationen der Produkte Propenoxid, 1-Methoxypropanol, 2-Methoxypropanol und 1,2-Propandiol, bei 94,5 %.

## Patentansprüche

1. Verfahren zur katalytischen Epoxidierung von Olefinen mit Wasserstoffperoxid in einem kontinuierlich durchströmten Reaktionssystem, wobei in dem Reaktionssytem eine gasförmige Phase, die ein Olefin enthält und eine flüssige Phase, die das Wasserstoffperoxid enthält, vorliegt,
dadurch gekennzeichnet, daß
die gasförmige Phase im Gegenstrom zur flüssigen Phase geführt wird.

2. Verfahren nach Anspruch 1,
dadurch gekennzeichnet, daß
das Reaktionssystem aus einem oder mehreren in Reihe oder parallel geschalteten Strömungsrohren ausgewählt ist.

3. Verfahren nach Anspruch 2,
dadurch gekennzeichnet, daß
das Reaktionssystem mehrere in Reihe geschaltete Strömungsrohre aufweist, wobei die Stoffströme von flüssiger Phase und gasförmiger Phase innerhalb eines Strömungsrohres entweder im Gleichstrom oder im Gegenstrom geführt werden und die Stoffströme zwischen den Strömungsrohren im Gegenstrom geführt werden.

4. Verfahren nach Anspruch 1,
dadurch gekennzeichnet, daß
das Reaktionssystem mehrere in Reihe geschaltete Reaktoren aufweist, die unabhängig von einander aus durchmischten Reaktoren und Strömungsrohren ausgewählt sind, wobei die Stoffströme von flüssiger Phase und gasförmiger Phase zwischen den Reaktoren im Gegenstrom geführt werden.

5. Verfahren nach einem der vorstehenden Ansprüche,
dadurch gekennzeichnet, daß
der Katalysator in der flüssigen Reaktionsphase suspendiert wird.

6. Verfahren nach einem der Ansprüche 2 oder 3,
dadurch gekennzeichnet, daß
der Katalysator in Form eines Festbetts eingesetzt wird.

7. Verfahren nach einem der vorstehenden Ansprüche,
dadurch gekennzeichnet, daß
als Katalysator ein titanhaltiger Zeolith eingesetzt wird.

8. Verfahren nach einem der vorstehenden Ansprüche,
dadurch gekennzeichnet, daß
das Olefin Propen ist.

9. Verfahren nach Anspruch 8,
dadurch gekennzeichnet, daß
ein Propenzuführstrom verwendet wird, der zusätzlich bis zu 15 Vol-% Propan enthält.
